# EUROPEAN PATENT APPLICATION

(11) **EP 4 442 240 A1**
(43) Date of publication of application: **09.10.2024**
(21) Application number: 22901584.7
(22) Date of filing: 28.10.2022
(51) Int. Cl.: A61K 8/02, A61Q 19/00, A23L 33/125, A23P 10/00, A61K 9/70, A61K 31/728

(54) **HYALURONIC ACID FILM WITH EXCELLENT PERCUTANEOUS ABSORPTION AND MANUFACTURING METHOD THEREFOR**

(30) Priority: 01.12.2021 KR 20210170203
(71) Applicant: Jinwoo Bio Co., Ltd., Giheung-gu Yongin-si, Gyeonggi-do 17111 (KR)
(72) Inventor: YOON, Hee Ju, Gyeonggi-do 17106 (KR); KWEON, Dong Keon, Yongin-si Gyeonggi-do 16918 (KR); HONG, Joo Yeon, Guri-si, Gyeonggi-do 11920 (KR); KWON, Sin Hwan, Seoul 01386 (KR)
(74) Representative: Ter Meer Steinmeister & Partner
(86) International application number: PCT/KR2022/016646
(87) International publication number: WO 2023/101225

(57) **Abstract**

The present invention relates to a hyaluronic acid film and a manufacturing method therefor and, more particularly, to a hyaluronic acid film having excellent percutaneous absorption and a manufacturing method therefor. The hyaluronic acid film of the present invention is characterized by having a percutaneous absorption rate of 72 to 93%. Having ultra-low molecular weight hyaluronic acid as a main component, the hyaluronic acid film of the present invention is superb in percutaneous absorption rate and mechanical properties and the can be applied to various purposes such as cosmetic packs, edible films and medical device patches, health functional foods, etc.

## Description

### Technical Field

The present disclosure relates to a hyaluronic acid film and a method of manufacturing the same, and more specifically, to a hyaluronic acid film with an excellent transdermal absorption rate and a method of manufacturing the same.

### Background Art

A hyaluronic acid or a salt thereof is distributed evenly in the connective tissue, epithelium, and nervous tissue of the human body. A hyaluronic acid or a salt thereof is a biocompatible material working in various physiological activities. A hyaluronic acid or a salt thereof can contain a large amount of moisture and has excellent viscoelasticity, so a hyaluronic acid or a salt thereof has been proven to be effective in skin regeneration, moisturizing, elasticity maintenance, and wrinkle treatment. Accordingly, recently, the demand for anti-aging cosmetics, food, pharmaceuticals, and fillers for medical devices containing the material is rapidly increasing.

Most finished hyaluronic acid salt products currently on the market are in a liquid form and contain excessive water, raising concerns about the risk of microbial contamination. Therefore, when commercializing a hyaluronic acid salt, due to safety concerns, the hyaluronic acid salt is required to be prepared in sterile equipment, or preservatives are required to be used.

Therefore, there has been research to solidify a hyaluronic acid or a salt thereof in the forms of films and fibers rather than liquifying the hyaluronic acid or salt thereof.

A hyaluronic acid or a salt thereof can be divided into an ultra-small-sized molecule, a small-sized molecule, a medium-sized molecule, and a large-sized molecule depending on the molecular weight.

Japanese Patent Application Publication No. 2014-114355 discloses a technology for manufacturing a hyaluronic acid film using a medium-sized molecular hyaluronic acid having an average molecular weight in a range of 1×10⁵ to 4×10⁶ Da. Korean Patent Application Publication No. 2019-0106091 also discloses a technology for manufacturing a hyaluronic acid salt film using medium-sized molecular and large-sized molecular hyaluronic acid salts having a molecular weight in a range of 0.1 to 2.5 MDa.

Medium-sized molecular and large-sized-molecular hyaluronic acid salts have excellent skin coating effects and mechanical properties, making it easy to manufacture a film, but the salts have difficulty penetrating and being absorbed into the skin.

Meanwhile, the lower the molecular weight of a hyaluronic acid or a salt thereof, the easier it is for the hyaluronic acid or salt thereof to penetrate and be absorbed into the skin, but the mechanical properties thereof are poor, making film manufacturing difficult.

In the meanwhile, Korea Patent Application Publication No. 2019-0138907 disclosed a technology and described that a hyaluronic acid film having a molecular weight in a range of 500 to 5,000,000 Da can be manufactured. However, in the embodiments, a specified molecular weight was not proposed. In fact, when a hyaluronic acid or a salt thereof had a molecular weight in a range of 2,000 Da or lower, a film was not manufactured. In the case of a hyaluronic acid or a salt thereof having a molecular weight in a range of 2,000 to 1×10⁴ Da, a film was formed, but the surfaces thereof were cracked, reducing marketability. When the molecular weight thereof was beyond 3.0×10⁴ Da, a film with an even surface was formed. However, a hyaluronic acid film having a molecular weight of 3.0×10⁴ Da or more had the problem of having a low transdermal absorption rate.

Accordingly, the present inventors attempted to manufacture a hyaluronic acid film that is easy to penetrate and be absorbed into the skin. As a result of their efforts, it was confirmed that when adding a medium-sized molecular hyaluronic acid salt having a molecular weight in a range of 1×10⁵ to 1×10⁶ Da to an ultra-small-sized molecular hyaluronic acid having a molecular weight in a range of 2,000 to 1×10⁴ Da, a hyaluronic acid film with an excellent transdermal absorption rate can be uniformly manufactured in large quantities, thereby leading to this disclosure.

### Disclosure

### Technical Problem

One objective of the present disclosure is to provide a hyaluronic acid film with an excellent transdermal absorption rate.

Another objective of the present disclosure is to provide a method of manufacturing a hyaluronic acid film with an excellent transdermal absorption rate.

Yet another objective of the present disclosure is to provide a mask pack, an edible film, and a patch for medical devices, all containing a hyaluronic acid film with an excellent transdermal absorption rate.

### Technical Solution

To achieve the objectives, the present disclosure provides a film including a hyaluronic acid and having a transdermal absorption rate in a range of 72% to 93%.

In the present disclosure, the hyaluronic acid includes 84.0% to 98.5% by weight of hyaluronic acid having a molecular weight in a range of 2,000 to 1×10⁴ Da and 1.5% to 16% by weight of hyaluronic acid having a molecular weight in a range of 1×10⁵ to 1×10⁶ Da.

In the present disclosure, the hyaluronic acid film has a thickness in a range of 0.01 to 1.5 mm.

The present disclosure also provides a method of manufacturing a hyaluronic acid film, the method including:
(a) preparing a hyaluronic acid solution by mixing hyaluronic acid having a molecular weight in a range of 2,000 to 1×10⁴ Da with hyaluronic acid having a molecular weight in a range of 1×10⁵ to 1×10⁶ Da and dissolving the mixture in a solvent;
(b) filtering the hyaluronic acid solution;
(c) introducing the filtered hyaluronic acid solution into an automatic applicator or a mold to cast a film to have a thickness range of 0.02 to 3 mm; and
(d) drying the cast film at a temperature in a range of 30°C to 60°C.

In the present disclosure, the weight ratio of the hyaluronic acid having a molecular weight of 2,000 to 1×10⁴ Da and the hyaluronic acid having a molecular weight of 1×10⁵ to 1×10⁶ Da is 84.0 to 98.5:1.5 to 16 in terms of % by weight.

In the present disclosure, the hyaluronic acid solution has a viscosity in a range of 2,000 to 25,000 cPs.

In the present disclosure, the hyaluronic acid solution has a pH level in a range of 2.5 to 3.5.

In the present disclosure, the hyaluronic acid is hyaluronic acid, a salt thereof, or a mixture thereof.

The present disclosure also provides a cosmetic mask pack, an edible film, and a patch for medical devices containing the hyaluronic acid film.

### Advantageous Effects

The hyaluronic acid film of the present disclosure has an ultra-small-sized molecular weight hyaluronic acid as a main ingredient thereof, so the hyaluronic acid film not only has an excellent transdermal absorption rate but also has excellent mechanical properties.

### Description of Drawings

FIG. 1 is a photograph of an ultra-small-sized molecular hyaluronic acid film;
FIG. 2 is a hyaluronic acid film containing a mixture of an ultra-small-sized molecular (8,000 Da) hyaluronic acid and a medium-sized molecular (1×10⁵ to 1×10⁶ Da) hyaluronic acid prepared according to one embodiment of the present disclosure;
FIG. 3 is a photograph related to the microbial stability evaluation of a hyaluronic acid film manufactured according to another embodiment of the present disclosure; and
FIG. 4 is a photograph of a hyaluronic acid film prepared without filtering a hyaluronic acid solution.

### Best Mode

The lower the molecular weight of a hyaluronic acid or a salt thereof, the easier it is for the hyaluronic acid or salt thereof to penetrate and be absorbed into the skin, but the mechanical properties thereof are poor, making film manufacturing difficult.

Korea Patent Application Publication No. 2019-0138907 disclosed a technology and described that a hyaluronic acid film having a molecular weight in a range of 500 to 5,000,000 Da can be manufactured. However, as a result of carrying out the method described in the specification, a film was not manufactured when the hyaluronic acid or salt thereof had a molecular weight in a range of 2,000 Da or lower. In the case of a hyaluronic acid or a salt thereof having a molecular weight in a range of 2,000 to 1×10,000 Da, a film was formed, but the surfaces thereof were cracked, reducing marketability. When the molecular weight thereof is 3.0×10⁴ Da or more, a film with an even surface is formed. However, a hyaluronic acid film having a molecular weight of 3×10⁴ Da or more has a low transdermal absorption rate, and thus, there is a problem in that the various physiological activities of the hyaluronic acid are not properly delivered to the skin.

In the present disclosure, the inventors sought to confirm that when adding a medium-sized molecular hyaluronic acid having a molecular weight in a range of 1×10⁵ to 1×10⁶ Da to an ultra-small-sized molecular hyaluronic acid having a molecular weight of 2,000 to 1×10⁴ Da, a hyaluronic acid film with an excellent transdermal absorption rate can be uniformly manufactured in large quantities.

In the present disclosure, an ultra-small-sized molecular hyaluronic acid and a medium-sized molecular hyaluronic acid were mixed and dissolved in purified water to prepare a hyaluronic acid solution having a viscosity in a range of 2,000 to 25,000 cPs (25°C). The solution was introduced into an automatic applicator or a mold and dried at a temperature in a range of 30°C to 60°C.

As a result, it was confirmed that a hyaluronic acid film with uniform surfaces could be manufactured in large quantities and that the manufactured film including the hyaluronic acid had a transdermal absorption rate in a range of 72% to 93%.

Accordingly, in one aspect, the present disclosure relates to a film including a hyaluronic acid and having a transdermal absorption rate in a range of 72% to 93%.

The hyaluronic acid includes 84.0% to 98.5% by weight of hyaluronic acid having a molecular weight in a range of 2,000 to 1×10⁴ Da and 1.5% to 16% by weight of hyaluronic acid having a molecular weight in a range of 1×10⁵ to 1×10⁶ Da.

In other words, since the hyaluronic acid film of the present disclosure cannot be formed with only the ultra-small-sized molecular hyaluronic acid, a medium-sized molecular hyaluronic acid is used together to form the skeleton of the film.

In the present disclosure, the ultra-small-sized molecular hyaluronic acid has a molecular weight of 2,000-1×10⁴ Da. The small-sized molecular hyaluronic acid has a molecular weight in range of 1×10⁴ to 1×10⁵ Da. The medium-sized molecular hyaluronic acid has a molecular weight in a range of 1×10⁵ to 1×10⁶ Da.

When the hyaluronic acid having a molecular weight in a range of 2,000 to 1×10⁴ Da is contained in an amount of less than 84.0% by weight, a film is formed, but the transdermal absorption rate thereof is low. Meanwhile, when the hyaluronic acid having a molecular weight in a range of 2,000 to 1×10⁴ Da is contained in an amount of more than 98.5% by weight, a film is not formed.

In the present disclosure, the hyaluronic acid film has a thickness in a range of 0.01 to 1.5 mm.

In the present disclosure, the hyaluronic acid may be hyaluronic acid, a salt thereof, or a mixture thereof. In addition, the hyaluronic acid salt is a salt bound to a hyaluronic acid. The hyaluronic acid salt may include but is not limited to, sodium hyaluronate, calcium hyaluronate, and potassium hyaluronate.

In another aspect, the present disclosure relates to a method of manufacturing a hyaluronic acid film, the method including:
(a) preparing a hyaluronic acid solution by mixing a hyaluronic acid having a molecular weight in a range of 2,000 to 1×10⁴ Da with a hyaluronic acid having a molecular weight in a range of 1×10⁵ to 1×10⁶ Da and dissolving the mixture in a solvent;
(b) filtering the hyaluronic acid solution;
(c) introducing the filtered hyaluronic acid solution into an automatic applicator or a mold to cast a film to have a thickness range of 0.02 to 3 mm; and
(d) drying the cast film at a temperature in a range of 30°C to 60°C.

The weight ratio of the hyaluronic acid having a molecular weight in a range of 2,000 to 1×10⁴ Da and the hyaluronic acid having a molecular weight in a range of 1×10⁵ to 1×10⁶ Da is preferably 84.0 to 98.5:1.5 to 16 in terms of % by weight.

In the present disclosure, the hyaluronic acid solution, which is a mixture of the ultra-small-sized molecular weight and the medium-sized molecular weight hyaluronic acid, preferably has a viscosity in a range of 2,000 to 25,000 cPs (25°C). The viscosity of the solution may vary depending on HA molecular weight (Da), HA content, and ratio with solvent. When the viscosity is less than 2,000 cPs, there is difficulty in forming a film. When the viscosity is beyond 25,000 cPs, the viscosity is so high that there is a risk that the film surface may become uneven when the film is cast to have a thickness range of 0.02 to 3 mm.

Before introducing the mixed hyaluronic acid solution into an automatic applicator or a mold, it is desirable to filter the solution to remove air bubbles and insoluble matter. The filtration may be performed by filtering the solution through a sieve (sieve size: 0.1 mm).

When the filtration is not performed, cracks occur around air bubbles or insoluble matter during film drying, resulting in a defective film.

In the present disclosure, the solvent is used to dissolve a hyaluronic acid or a salt thereof. Purified water or ethanol aqueous solution can be used as the solvent, and the ethanol aqueous solution preferably has ethanol contained in an amount of 0.01% to 10% by volume.

Generally, the hyaluronic acid salt has a pH level in a range of 5.0 to 7.0. A hyaluronic acid solution is vulnerable to microbial contamination, so to prevent this, antibacterial agents such as ethanol are required to be added. When using a hyaluronic acid having a pH level in a range of 2.5 to 3.5, the effect of preventing microbial contamination is excellent.

The filtered hyaluronic acid solution is injected into an automatic applicator or a mold, and then a film of the hyaluronic acid solution is cast to have a thickness range of 0.02 to 3 mm. Next, the cast film is dried at a temperature in a range of 30°C to 60°C. Through this, a hyaluronic acid film having a thickness in a range of 0.01 to 1.5 mm may be manufactured.

When the casting thickness is less than 0.02 mm, film formation is difficult. When the casting thickness is above 3 mm, the thickness is not maintained due to the flowability of the solution. Ultimately, a hyaluronic acid film having a thickness in a range of 0.01 mm to 1.5 mm may be manufactured.

Drying of the cast film is preferably performed at a temperature in a range of 30°C to 60°C for 6 to 24 hours. The drying can be performed using various drying methods without particular limitations as long as the drying is performed at a temperature in a range of 30°C to 60°C.

In yet another aspect, the present disclosure relates to a cosmetic mask pack, an edible film, and a patch for medical devices containing the hyaluronic acid film.

### Mode for Disclosure

Hereinafter, the present disclosure will be described in more detail through examples. These examples are intended solely to illustrate the present disclosure. It will be apparent to those skilled in the art that the scope of the present disclosure is not to be construed as limited by these examples.

### Example 1: Manufacturing of hyaluronic acid films according to the conventional art

Hyaluronic acid films were manufactured by the method described in Korean Patent Application Publication No. 2019-0138907. That is, glass was used as a support and heated to a temperature of 60°C for 2 hours. A hyaluronic acid solution was prepared by dissolving 17% by weight of hyaluronic acid based on the total weight of the hyaluronic acid solution in distilled water. The dissolved hyaluronic acid solution was poured to a thickness of 500 um on the heated support (glass) to make a coating thereon. The coated support was dried at a temperature of 60°C for 2 hours, and peeling was conducted to prepare a hyaluronic acid film having a thickness of 80 um. At this time, the molecular weights of the hyaluronic acid used were 500 Da, 2,000 Da, and 1×10⁵ Da, respectively.

As a result, it was confirmed that when the molecular weight of the hyaluronic acid was 500 Da, a film was not formed. In addition, when the molecular weight of the hyaluronic acid was 2,000 Da, a film was formed, but the film was not smooth, and when the molecular weight of the hyaluronic acid was 1×10⁵ Da, a film with a smooth surface was formed.

### Example 2: Manufacturing of hyaluronic acid films

### Examples 2-1 to 2-6: Manufacturing of ultra-small-sized molecular hyaluronic acid films

A hyaluronic acid salt solution (100 to 20,000 cPs) was prepared by dissolving an ultra-small-sized molecular hyaluronic acid salt (10% to 50% by volume) having a molecular weight in a range of 500 to 1×10⁵ Da in a 0.01% to 10% aqueous ethanol solution. Next, by adjusting the applicator of an automatic applicator, a film was cast to have a thickness range of 0.02 to 3 mm. Afterward, the cast film was dried in a hot air dryer at a temperature of 40°C for 20 minutes to 24 hours to prepare a hyaluronic acid salt film of the hyaluronic acid salt having a thickness in a range of 0.01 to 1.5 mm.

**[Table 1]**

| Example | HA molecular weight (Da) | Viscosity (cPs) |
|---|---|---|
| 2-1 | 500 | 100 |
| 2-2 | 2,000 | 400 |
| 2-3 | 8,000 | 933 |
| 2-4 | 10,000 | 1,500 |
| 2-5 | 30,000 | 3, 633 |
| 2-6 | 100,000 | 18,000 |

As a result, similar to the results prepared by the conventional art in Example 1, when the molecular weight was 500 Da, a film was not formed. In addition, similar to the results prepared by the conventional art in Example 1, when the molecular weight was 2,000 Da, 8,000 Da, and 10,000 Da, a film was formed, but the film was easily cracked and broken, and when the molecular weight was 3×10⁴ Da or higher, a film with a smooth surface was formed.

### Examples 2-7 to 2-23: Manufacturing of hyaluronic acid films using ultra-small-sized and medium-sized molecules

As shown in Table 2 below, a medium-sized molecular hyaluronic acid salt (pH level of 6.0 to 7.0) having a molecular weight in a range of 1×10⁵ to 1×10⁶ Da and an ultra-small-sized molecular hyaluronic acid salt (pH level of 6.0 to 7.0) having a molecular weight in a range of 2,000 to 10,000 Da were mixed. Next, the mixture was dissolved in purified water to prepare a hyaluronic acid salt solution having a viscosity in a range of 2,000 to 25,000 cPs. The prepared solution was filtered through a sieve (sieve size: 0.1 mm). Afterward, by adjusting the applicator of the automatic applicator, a film of the hyaluronic acid salt solution was cast to have a thickness range of 0.02 to 3 mm, and then the film was dried in a hot air dryer at a temperature of 40°C for 20 minutes to 24 hours to prepare a hyaluronic acid salt film having a thickness range of 0.01 to 1.5 mm.

**[Table 2]**

| Example | Ultra-small-sized molecule | | Medium-sized molecule | | HA solution Viscosity (cPs) | HA film thickness (mm) |
|---|---|---|---|---|---|---|
| | HA molecular weight (Da) | Weight % | HA molecular weight (Da) | Weight % | | |
| 2-7 | 2,000 | 98.3 | 1 × 10⁵ | 1.7 | 2,000 | 0.01 |
| 2-8 | 2,000 | 98.3 | 1 × 10⁵ | 1.7 | 2,000 | 1.5 |
| 2-9 | 2,000 | 94 | 1 × 10⁶ | 6 | 3,500 | 0.025 |
| 2-10 | 2,000 | 100 | - | - | 400 | - |
| 2-11 | 5,000 | 94 | 1 × 10⁶ | 6 | 6,700 | 0.025 |
| 2-12 | 5,000 | 100 | - | - | 733 | - |
| 2-13 | 8,000 | 94 | 1 × 10⁶ | 6 | 10,000 | 0.025 |
| 2-14 | 10,000 | 94 | 1 × 10⁶ | 6 | 12,333 | 0.025 |
| 2-15 | 10,000 | 100 | - | - | 1,500 | - |
| 2-16 | 10,000 | 98.3 | 1 × 10⁶ | 1.7 | 6,700 | 0.025 |
| 2-17 | 10,000 | 95.8 | 1 × 10⁶ | 4.2 | 10,633 | 0.025 |
| 2-18 | 10,000 | 90 | 1 × 10⁶ | 10 | 17,600 | 0.025 |
| 2-19 | 10,000 | 84.6 | 1 × 10⁶ | 15.4 | 24,700 | 0.025 |
| 2-20 | 10,000 | 84.6 | 1 × 10⁶ | 15.4 | 24,700 | 1.5 |
| 2-21 | 10,000 | 84.6 | 1 × 10⁶ | 15.4 | 24,700 | - |
| 2-22 | 10,000 | 80 | 1 × 10⁶ | 20 | 29,700 | - |
| 2-23 | 10,000 | 75 | 1 × 10⁶ | 25 | 37,333 | - |

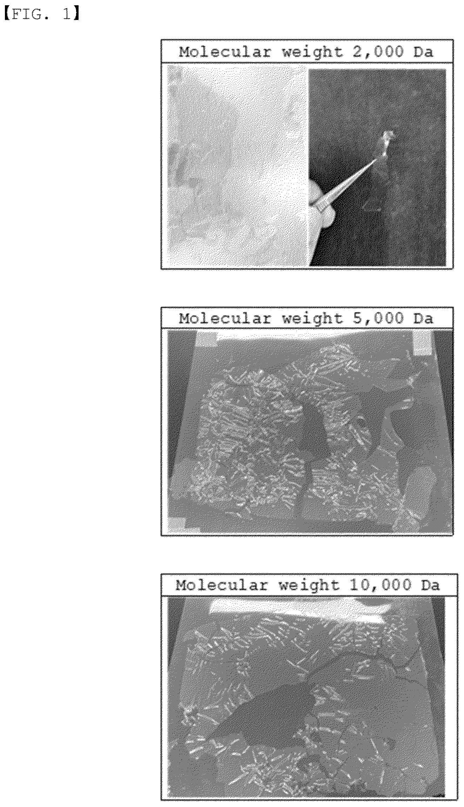

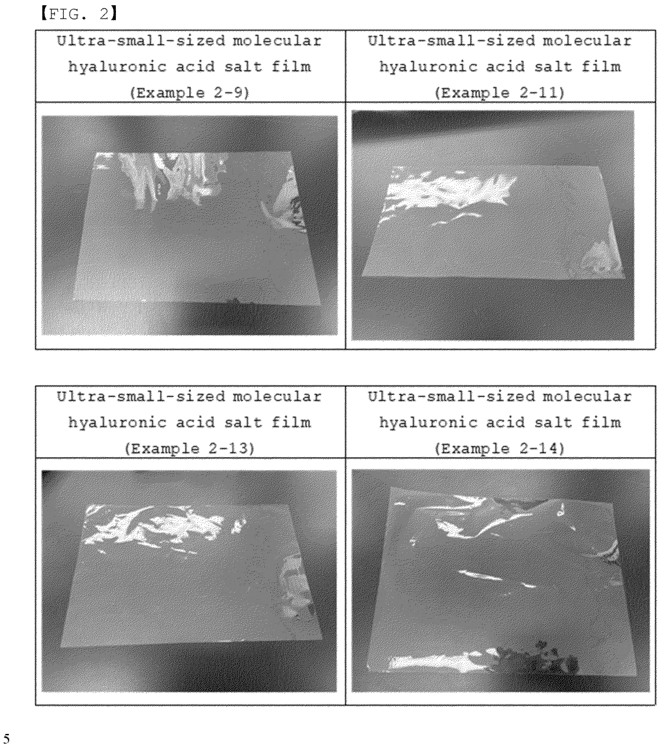

From FIG. 1, it was confirmed that film formation was impossible since the surface of the film was cracked and had an uneven appearance when the ultra-small-sized molecular hyaluronic acid salt having a molecular weight in a range of 2,000 to 1×10⁴ Da was used alone. Meanwhile, it was confirmed that mass production of a film with a uniform surface was possible when mixing the ultra-small-sized molecular hyaluronic acid salt and the medium-sized molecular hyaluronic acid salt at a specific ratio (see FIG. 2).

From Table 2 above, it was confirmed that film formation was difficult when the hyaluronic acid salt solution had a viscosity of less than 2,000 cPs. In addition, it was confirmed that when the hyaluronic acid salt solution had a viscosity of 25,000 cPs or more, the viscosity was high, so the casting of the film with such hyaluronic acid salt solution to be in a thickness range of 0.02 to 3 mm was impossible using an automatic applicator.

Additionally, it was confirmed that when film casting was conducted by setting the casting thickness to 3.5 mm, the thickness of the hyaluronic acid salt solution was not maintained, and the solution was spread, making film casting impossible.

### Examples 2-24 to 2-26: Manufacturing of hyaluronic acid films depending on pH level

Hyaluronic acid films were manufactured in the same manner as Examples 2-9, 2-11, and 2-13 except that a hyaluronic acid having a pH level in a range of 2.5 to 3.5 was used instead of the hyaluronic acid salt having a pH level in a range of 6.0 to 7.0, and the stability of the films against microorganisms was evaluated.

**[Table 3]**

| Example | Ultra-small-sized molecule | | Medium-sized molecule | | | pH |
|---|---|---|---|---|---|---|
| | HA molecular weight (Da) | Weight % | HA molecular weight (Da) | Weight % | HA solution Viscosity (cPs) | |
| 2-24 | 2,000 | 94 | 1 × 10⁶ | 6 | 3,500 | 2.5 |
| 2-25 | 5,000 | 94 | 1 × 10⁶ | 6 | 6,700 | 3.0 |
| 2-26 | 8,000 | 94 | 1 × 10⁶ | 6 | 10,000 | 3.5 |

The manufactured hyaluronic acid and hyaluronic acid salt films were each dissolved in a sterile saline solution at 1% volume. The lysates were applied by 1 ml to 3M petrifilm and incubated at a temperature of 37°C for 24 hours. Microbial contamination was checked, and the results are shown in Table 4.

**[Table 4]**

| Example | Microbial count (cfu/g) |
|---|---|
| 2-9 | 400 |
| 2-11 | 100 |
| 2-13 | 400 |
| 2-24 | 0 |
| 2-25 | 0 |
| 2-26 | 0 |

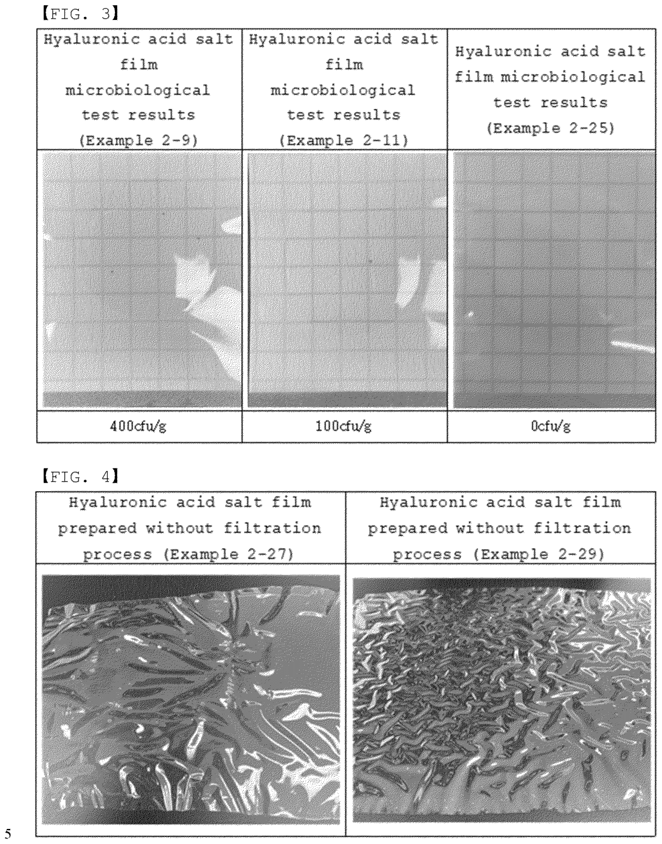

From Table 4, it was confirmed that when using the hyaluronic acid salt having a pH level in a range of 6.0 to 7.0, contamination with microorganisms could occur during the film manufacturing process, and when using the hyaluronic acid having a pH level in a range of 2.5 to 3.5, contamination with microorganisms did not occur (see FIG. 3).

### Examples 2-27 to 2-29: Manufacturing of hyaluronic acid films with or without filtration

Hyaluronic acid salt films were manufactured in the same manner as Examples 2-9, 2-11, and 2-13 except that the hyaluronic acid salt solution was not filtered and films were cast, and the quality of the hyaluronic acid films was confirmed.

**[Table 5]**

| Example | Film quality |
|---|---|
| 2-9 | Good |
| 2-11 | Good |
| 2-13 | Good |
| 2-27 | Generation of bubbles and wrinkles |
| 2-28 | Generation of bubbles and wrinkles |
| 2-29 | Generation of bubbles and wrinkles |

From Table 5 and FIG. 4, it was confirmed that when the hyaluronic acid solution was filtered through a sieve (sieve size: 0.1 mm) before casting, no bubbles were generated, and good films without impurities were formed, but when the hyaluronic acid solution was not filtered, bubbles were generated, or impurities were contained in the films.

### Example 3: Transdermal absorption rate evaluation

The transdermal absorption rates of the large-sized molecular hyaluronic acid salt films and the ultra-small-sized molecular hyaluronic acid salt films manufactured in the examples were compared. The large-sized molecular hyaluronic acid salt films were manufactured in the same manner as in Example 2-9 except that 100% by weight of hyaluronic acid salt having a molecular weight of 1.2 MDa was used, and the transdermal absorption rates were measured.

According to the "Guideline for in vitro skin absorption method," skin penetration tests were conducted under sink conditions using a Franz diffusion cell (effective area: 0.64 cm², volume of storage compartment: 5 ul) equipment (Logan Instruments, New Jersey, USA).

The aqueous phase used in the skin permeation tests was phosphate-buffered saline (PBS) at a pH level of 7.4. A prepared epidermal layer of human skin (1.5 cm × 1.5 cm) was placed on a receptor chamber with the stratum corneum of the epidermal layer upward. The stratum corneum was covered with a donor chamber, and then the epidermal layer, receptor chamber, and donor chamber were secured with a clamp. Next, the aqueous phase filled a Franz diffusion cell, and the aqueous phase was maintained at a temperature of 32 ± 1°C, and then by uniformly applying 100 µl of the hyaluronic acid film solution to the donor area, permeation tests were conducted. At 1 hour, 3 hours, 6 hours, 9 hours, 12 hours, and 24 hours after the permeation tests, 5 ml of the aqueous phase was collected and analyzed, and supplemented with fresh phosphate-buffered saline solution.

**[Table 6]**

| Example | HA molecular weight and content | HA film thickness (mm) | Transdermal absorption rate |
|---|---|---|---|
| 3-1 | 1.2×10⁶ Da | 0.025 | 33.12% |
| 2-4 | 1×10⁴ Da | 0.025 | 79.4% |
| 2-5 | 3×10⁴ Da | 0.025 | 63.1% |
| 2-6 | 1×10⁵ Da | 0.025 | 43.70% |
| 2-7 | Ultra-small-sized molecular weight (2,000 Da) 98.3% by weight, medium-sized molecular weight (1×10⁵ Da) 1.7% by weight | 0.01 | 93.63% |
| 2-8 | Ultra-small-sized molecular weight (2,000 Da) 98.3% by weight, medium-sized molecular weight (1×10⁵ Da) 1.7% by weight | 1.5 | 89.03% |
| 2-9 | Ultra-small-sized molecular weight (2,000 Da) 94% by weight, medium-sized molecular weight (1×10⁶ Da) 6% by weight | 0.025 | 92.01% |
| 2-11 | Ultra-small-sized molecular weight (5,000 Da) 94% by weight, medium-sized molecular weight (1×10⁶ Da) 6% by weight | 0.025 | 89.01% |
| 2-13 | Ultra-small-sized molecular weight (8,000 Da) 94% by weight, medium-sized molecular weight (1×10⁶ Da) 6% by weight | 0.025 | 83.33% |
| 2-14 | Ultra-small-sized molecular weight (10,000 Da) 94% by weight, medium-sized molecular weight (1×10⁶ Da) 6% by weight | 0.025 | 75.01% |
| 2-16 | Ultra-small-sized molecular weight (10,000 Da) 98.3% by weight, medium-sized molecular weight (1×10⁶ Da) 1.7% by weight | 0.025 | 77.70% |

| | | | |
|---|---|---|---|
| 2-17 | Ultra-small-sized molecular weight (10,000 Da) 95.8% by weight, medium-sized molecular weight (1×10⁶ Da) 4.2% by weight | 0.025 | 76.93% |
| 2-18 | Ultra-small-sized molecular weight (10,000 Da) 90% by weight, medium-sized molecular weight (1×10⁶ Da) 10% by weight | 0.025 | 75.80% |
| 2-19 | Ultra-small-sized molecular weight (10,000 Da) 84.6% by weight, medium-sized molecular weight (1×10⁶ Da) 15.4% by weight | 0.025 | 74.01% |
| 2-20 | Ultra-small-sized molecular weight (10,000 Da) 84.6% by weight, medium-sized molecular weight (1×10⁶ Da) 15.4% by weight | 1.5 | 71.50% |

From Table 6, it was confirmed that when using the large-sized molecular hyaluronic acid salt having a molecular weight of 1.2×10⁶ Da, the transdermal absorption rate of the films was 33.12%. Meanwhile, when mixing and using the ultra-small-sized molecular and the medium-sized molecular hyaluronic acid, the transdermal absorption rates of the hyaluronic acid films manufactured by the mixture were in a range of 71.50% to 93.63%. Thus, the hyaluronic acid films manufactured by the mixture had a higher transdermal absorption rate. In particular, it was confirmed that as the molecular weight decreased, the transdermal absorption rate significantly increased.

As described above, specific parts of the present disclosure have been described in detail. It will be clear to those skilled in the art that these specific techniques are merely preferred examples and do not limit the scope of the present disclosure. Accordingly, the substantial scope of the present disclosure will be defined by the appended claims and their equivalents.

### Industrial Applicability

The hyaluronic acid film of the present disclosure has an ultra-small-sized molecular weight hyaluronic acid as a main ingredient thereof, so the hyaluronic acid film not only has an excellent transdermal absorption rate but also has excellent mechanical properties. Thus, the hyaluronic acid film can be applied for various purposes such as a cosmetic pack, a patch for medical devices, and a health functional food.

## Claims

1. A film comprising a hyaluronic acid and having a transdermal absorption rate in a range of 72% to 93%.

2. The hyaluronic acid film of claim 1, wherein the hyaluronic acid comprises 84% to 98.5% by weight of hyaluronic acid having a molecular weight in a range of 2,000 to 1×10⁴ Da and 1.5% to 16% by weight of hyaluronic acid having a molecular weight in a range of 1×10⁵ to 1×10⁶ Da.

3. The hyaluronic acid film of claim 1, having a thickness in a range of 0.01 to 1.5 mm.

4. The hyaluronic acid film of any one of claims 1 to 3, wherein the hyaluronic acid comprises the hyaluronic acid, a salt thereof, or a mixture thereof.

5. A method of manufacturing a hyaluronic acid film, the method comprising:
(a) preparing a hyaluronic acid solution by mixing a hyaluronic acid having a molecular weight in a range of 2,000 to 1×10⁴ Da with a hyaluronic acid having a molecular weight in a range of 1×10⁵ to 1×10⁶ Da and dissolving the mixture in a solvent;
(b) filtering the hyaluronic acid solution;
(c) introducing the filtered hyaluronic acid solution into an automatic applicator or a mold to cast a film having a thickness range of 0.02 to 3 mm; and
(d) drying the cast film at a temperature in a range of 30°C to 60°C.

6. The method of claim 5, wherein a weight ratio of the hyaluronic acid having a molecular weight of 2,000 to 1×10⁴ Da and the hyaluronic acid having a molecular weight of 1×10⁵ to 1×10⁶ Da is 84.0 to 98.5:1.5 to 16 in terms of % by weight.

7. The method of claim 5, wherein the hyaluronic acid solution has a viscosity in a range of 2,000 to 25,000 cPs.

8. The method of claim 5, wherein the hyaluronic acid solution has a pH level in a range of 2.5 to 3.5.

9. The method of any one of claims 5 to 8, wherein the hyaluronic acid comprises a hyaluronic acid, a salt thereof, or a mixture thereof.

10. A cosmetic mask pack comprising the hyaluronic acid film of claim 1.

11. An edible film comprising the hyaluronic acid film of claim 1.

12. A patch for a medical device, the patch comprising the hyaluronic acid film of claim 1.
